(19) Europäisches Patentamt
European Patent Office
Office européen des brevets

(11) EP 2 830 612 B1

(12) **EUROPEAN PATENT SPECIFICATION**

(45) Date of publication and mention of the grant of the patent:
**04.05.2016 Bulletin 2016/18**

(21) Application number: **13712244.6**

(22) Date of filing: **25.03.2013**

(51) Int Cl.:
***A61K 31/19*** *(2006.01)* ***C12R 1/00*** *(2006.01)*
***A61P 1/00*** *(2006.01)* ***A61P 29/00*** *(2006.01)*
***A61P 35/00*** *(2006.01)* ***C12N 1/20*** *(2006.01)*
***C12P 7/40*** *(2006.01)* ***A23L 29/00*** *(2016.01)*
***A23L 5/00*** *(2016.01)* ***A23L 33/10*** *(2016.01)*

(86) International application number:
**PCT/EP2013/056268**

(87) International publication number:
**WO 2013/144083 (03.10.2013 Gazette 2013/40)**

(54) **4-OXO-2-PENTENOIC ACID AND THE HEALTH OF THE DIGESTIVE TRACT**
4-OXO-2-PENTAENSÄURE UND DIE GESUNDHEIT DES VERDAUUNGSTRAKTS
ACIDE 4-OXO-2-PENTÉNOÏQUE ET SANTÉ DES VOIES DIGESTIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2012 EP 12162366**

(43) Date of publication of application:
**04.02.2015 Bulletin 2015/06**

(73) Proprietor: **Nestec S.A.**
**1800 Vevey (CH)**

(72) Inventors:
- **ARCE VERA, Francia, Jacqueline**
  **CH-1000 Lausanne 26 (CH)**
- **BOURQUI, Bertrand**
  **CH-1489 Murist (CH)**
- **BUETLER, Timo**
  **CH-8051 Zurich (CH)**
- **DUBOUX, Stéphane**
  **CH-1162 St-Prex (CH)**
- **FOATA, Francis**
  **CH-1012 Lausanne (CH)**
- **GUY, Philippe, Alexandre**
  **CH-1522 Lucens (CH)**
- **PAGE, Nicolas**
  **CH-1012 Lausanne (CH)**
- **REZZI, Serge, André, Dominique**
  **CH-1623 Semsales (CH)**

(74) Representative: **Couzens, Patrick John**
**Nestec S.A.**
**CT-IAM**
**Avenue Nestlé 55**
**1800 Vevey (CH)**

(56) References cited:
**WO-A1-93/13076 US-A1- 2005 119 276**

- **PASPARAKIS M: "IKK/NF-[kappa]B signaling in intestinal epithelial cells controls immune homeostasis in the gut", MUCOSAL IMMUNOLOGY 2008 GB LNKD-DOI:10.1038/MI.2008.53, vol. 1, no. SUPPL. 1, 2008, pages S54-S57, XP002677708, ISSN: 1933-0219**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 830 612 B1

## Description

The present invention relates generally to compositions with a health benefit. In particular, the invention relates to the field of inflammatory disorders of the digestive tract, consisting of ileitis, colitis, rectal inflammation, pharyngitis, leaky gut syndrome, irritable bowel syndrome and inflammatory bowel disease.

Inflammatory disorders of the digestive tract can have a wide range of consequences, from short term illness or discomfort right through to major lifelong disability and death. Longterm repeated gastrointestinal inflammation not only influences the quality of life of patients but also increases the risk of fibrosis and carcinoma in the intestinal tract. The incidence of inflammatory bowel disease is rising in developing countries around the world and approximately 5 million people worldwide are currently affected.

The current treatments for inflammatory disorders of the digestive tract are based on the use of five main classes of drugs : non-specific anti-inflammatory drugs such as 5-aminosalicylic acid or glucocorticoids; antimetabolites such as azathioprine or 6-mercaptopurine; monoclonal antibodies such as infliximab; or antibiotics. However, in some circumstances these drugs can exhibit side effects and so it would be desirable to have additional compositions available that treat or prevent inflammatory disorders of the digestive tract without the drawbacks of those described in the prior art. In particular, it would be highly desirable to find an effective composition whose active ingredient is obtained from a natural source.

Consequently, it was the object of the present invention to improve the state of the art and in particular to provide an alternative composition that can be used to treat or prevent inflammatory disorders of the digestive tract as defined in the claims.

The inventors were surprised to see that the object of the present invention could be achieved by the subject matter of the independent claim. The dependent claims further develop the idea of the present invention.

Accordingly, the present invention provides a composition comprising 4-oxo-2-pentenoic acid for use in the treatment or prevention of inflammatory disorders of the digestive tract selected from the group consisting of ileitis, colitis, pharyngitis, leaky gut syndrome, irritable bowel syndrome, rectal inflammation, inflammatory bowel disease and combinations thereof. The composition is not a pharmaceutical.

The present invention also provides the use of 4-oxo-2-pentenoic acid in the preparation of a composition for the treatment or prevention of inflammatory disorders of the digestive tract selected from the group consisting of ileitis, colitis, pharyngitis, leaky gut syndrome, irritable bowel syndrome, rectal inflammation, inflammatory bowel disease and combinations thereof.

"Treatment" within the scope of the present invention refers to reduction, inhibition, alleviation or amelioration.

4-oxo-2-pentenoic acid has the CAS number 4743-82-2 and the following formula

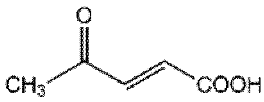

Nuclear factor (erythroid-derived 2)-like 2, also called Nrf2, is a critical regulator of inflammation (C.L.L. Saw et al., Expert Opinion on Therapeutic Targets, 15, 281-295 (2011)). Nrf2 resides in the cytosol and is bound to an inhibitor Keapl. When bound to Keap1, Nrf2 is also rapidly degraded by the proteasome hence its low basal concentration. Upon activation by stressors (for example nitric oxide, growth factors or heavy metals) Nrf2 is released from Keap1. Nrf2 concentration increases and it translocates into the nucleus. Nrf2 then binds to the antioxidant-response element (ARE) that is present in the promoter region of genes encoding many antioxidant enzymes (Kensler TW et al., Annu Rev Pharmacol Toxicol 2007;47:89-116). Recent studies have demonstrated that Nrf2-ARE signaling is involved in attenuating inflammation-associated pathogenesis, such as gastritis and colitis. It is likely that the cytoprotective function of genes targeted by Nrf2 may repress the induction of pro-inflammatory genes (J. Kim et al., Mutation Research - Fundamental and Molecular Mechanisms of Mutagenesis, 690, 12-23 (2010)).

Activation of Nrf2 by a number of compounds has been described. Polyphenols such as curcumin (US2009/0042980), resveratrol (Chen CY et al., Biochem Biophys Res Commun 2005 Jun 17;331(4):993-1000), sulphoraphane (F. Elbarbry et al., Journal of Medical Plants Research, 5, 473-484, (2011)) and quercitin (Tanigawa S et al., Free Radic Biol Med 2007 Jun 1;42(11):1690-703) have been reported to activate Nrf2. The inventors were surprised to find that 4-oxo-2-pentenoic acid also activates Nrf2. The very low aqueous solubility of curcumin, resveratrol, sulphoraphane and quercitin affects their bio-availability. 4-oxo-2-pentenoic acid, by contrast, has good aqueous solubility.
Activation of the transcription factor nuclear factor κB (NF-κB) is implicated in a host of inflammatory conditions including colitis and inflammatory bowel disease. (M. Pasparakis, Mucosal Immunology, 1, S54-S57 (2008)). NF-κB consists of homodimers and heterodimers of Rel proteins. The predominant transactivating form of NF-κB consists of p65 and p50

heterodimers. The activation of NF-kB involves the phosphorylation and subsequent proteolytic degradation of the inhibitory protein IκB by specific IκB kinases. The free NF-κB passes into the nucleus, where it binds to κB sites in the promoter region of numerous genes involved in inflammation.
Inhibitors of NF-κB have been identified, such as glucocorticoids (Adcock et al., American Journal of Physiology - Cell Physiology, 268, C331-C338 (1995)), but glucocorticoids have endocrine and metabolic side effects when given systemically. Heparin has also been reported to inhibit NF-κB (WO200119376), but it has the potential side-effect of causing heparin-induced thrombocytopenia.

WO9313076 discloses the use of 4-oxo-2-pentenoic acid for the treatment of inflammatory and ulcerative diseases of the esophagus, stomach and duodenum. It is suggested in said document that 4-oxo-2-pentenoic acid has gastroprotective effects.

The inventors investigated whether 4-oxo-2-pentenoic acid inhibits NF-κB activation. Using human colonic cells and human monocytes/macrophages they found that 4-oxo-2-pentenoic acid inhibits NF-κB activation under pro-inflammatory stresses (LPS and hTNF-α).
The inventors were surprised to find that 4-oxo-2-pentenoic acid was obtainable from natural sources, e.g., from some heat treated bacterial strains. For example, bacterial preparations of *Bifidobacterium breve* CNCM 1-3865 and *Bifidobacterium breve* ATCC 15700™ both yielded 4-oxo-2-pentenoic acid when heated for 6 hours at 90°C. 4-oxo-2-pentenoic acid was found to be in the soluble fraction after centrifuging and filtering the heat treated bacterial preparations.
*Bifidobacterium breve* CNCM 1-3865 was deposited with the COLLECTION NATIONALE DE CULTURES DE MICROORGANISMES (CNCM), INSTITUT PASTEUR, 25 rue du Docteur Roux, F-75724 PARIS Cedex 15, France, on November 15th, 2007. *Bifidobacterium breve* ATCC 15700™ can be obtained commercially, e.g., from the American type Culture Collection (ATCC), Manassas, Virginia, USA, under the trademark ATCC 15700.
Consequently the present invention relates in part to a composition comprising 4-oxo-2-pentenoic acid for use in the treatment or prevention of inflammatory disorders of the digestive tract selected from the group consisting of ileitis, colitis, pharyngitis, leaky gut syndrome, irritable bowel syndrome, rectal inflammation, inflammatory bowel disease and combinations thereof; wherein the composition is not a pharmaceutical. A pharmaceutical is a drug or medicine that is prepared or dispensed in pharmacies and used in medical treatment (<URL: www.thefreedictionary.com/ pharmaceutical/> [retrieved on 17-07-2012]). The present invention may be a food composition comprising 4-oxo-2-pentenoic acid for use in the treatment or prevention of inflammatory disorders of the digestive tract as defined in the claims. The digestive tract comprises the mouth, throat, esophagus, forestomachs (reticulum, rumen, omasum) of ruminants, the true stomach in all species, small intestine, large intestine and, in species such as humans which have them, the rectum and anus.

Ileitis, and colitis are inflammations of the ileum and large intestine respectively. Pharyngitis is an inflammation of the throat or pharynx.

Leaky gut syndrome, also called increased intestinal permeability, is a well-recognised and common diagnosis within the community of integrative doctors (B. Brom, South African Family Practice, 52, 314-316 (2010)). Health care practitioners who diagnose this syndrome explain that intestinal inflammation widens the junctions between the cells of the intestinal lining. Increased permeability stimulates classic hypersensitivity responses to foods and to components of the normal gut flora. Bacterial endotoxins, cell wall polymers and dietary gluten may cause "non-specific" activation of inflammatory pathways (L. Galland, (1995), viewed 26th October 2011, http://www.mdheal.org/leakygut.htm). Low grade fever, transient gut pain, and a sense of inability to absorb nutrients are some reported symptoms in otherwise undiagnosed patients.

Irritable bowel syndrome is a functional bowel disorder characterized by chronic abdominal pain, discomfort, bloating, and alteration of bowel habits in the absence of any detectable organic cause. Studies have described mucosal inflammation in irritable bowel syndrome patients and a disease correlation with intestinal infections (G. Barbara et al., Gut, 51 (SUPPL. 1), i41-i44 (2002)).

Inflammatory bowel disease is a group of gastrointestinal diseases characterized by chronic and periodic inflammation.

The major forms of inflammatory bowel disease are Crohn's disease and ulcerative colitis, although other forms of inflammatory bowel disease include collagenous colitis, lymphocytic colitis, ischaemic colitis, diversion colitis, Behçet's disease and indeterminate colitis. With the scale and severity of such disorders worldwide it is therefore advantageous to provide a composition to treat or prevent inflammatory disorders of the digestive tract such as ileitis, colitis, rectal inflammation, pharyngitis, leaky gut syndrome, irritable bowel syndrome and/or inflammatory bowel disease

In the present invention, the 4-oxo-2-pentenoic acid may be obtainable, for example obtained, from natural sources. Many people are concerned about the safety of materials industrially synthesised from chemical feedstock, especially when these materials are to be ingested, and prefer materials obtained from natural sources.

Surprisingly, the inventors found that some strains of bacteria provide a natural source of 4-oxo-2-pentenoic acid. In particular, the inventors have found that 4-oxo-2-pentenoic acid can be obtained from *Bifidobacterium breve* CNCM 1-3865 or *Bifidobacterium breve* ATCC 15700™ (the type strain for *Bifidobacterium breve*). It is particularly

advantageous to use bacteria as a source of 4-oxo-2-pentenoic acid as the production of large quantities of 4-oxo-2-pentenoic acid is feasible, for example using bioreactors. Accordingly, in the present invention the 4-oxo-2-pentenoic acid may be obtainable, for example obtained, from *Bifidobacterium breve* CNCM 1-3865 or *Bifidobacterium breve* ATCC 15700™.

The bacteria may be heat treated at about 60-180°C, preferably at about 80-160°C, for example at about 110-150°C in commercial production processes. The inventors found that heat treatment at these temperatures provided a satisfactory yield of 4-oxo-2-pentenoic acid within an acceptable time. Without wishing to be bound by theory it is understood that increasing the temperature of heat treatment increases the rate of formation of 4-oxo-2-pentenoic acid but also increases the rate of its degradation. Accordingly these temperatures give a good balance between the rate of formation of 4-oxo-2-pentenoic acid and its degradation.

Typical compositions comprising 4-oxo-2-pentenoic acid may comprise 4-oxo-2-pentenoic acid in an amount of at least 1 mg/kg of the composition. Generally, it is preferred if the composition comprises 4-oxo-2-pentenoic acid in an amount of at least 10 mg/kg of the composition, for example between 50 mg and 50 g per kg of the composition.

The optimum amount of 4-oxo-2-pentenoic acid to be administered can be easily determined by skilled artisans.

In therapeutic applications, compositions are administered in an amount sufficient to at least partially cure or arrest the symptoms of the disorder and/or its complications. An amount adequate to accomplish this is defined as "a therapeutic effective dose". Amounts effective for this purpose will depend on a number of factors known to those of skill in the art such as the severity of the disorder and the weight and general state of the patient. In prophylactic applications, compositions according to the invention are administered to a patient susceptible to or otherwise at risk of a particular disorder in an amount that is sufficient to at least partially reduce the risk of developing a disorder. Such an amount is defined to be "a prophylactic effective dose". Again, the precise amounts depend on a number of patient specific factors such as the patient's state of health and weight.

4-oxo-2-pentenoic acid may be administered in the framework of the present invention in a therapeutic effective dose and/or in a prophylactic effective dose. The composition of the present invention may be administered in a daily dose corresponding to between 2 μg and 20 mg of 4-oxo-2-pentenoic acid per kg of body weight, preferably between 20 μg and 2 mg of 4-oxo-2-pentenoic acid per kg of body weight, for example between 40 μg and 1 mg of 4-oxo-2-pentenoic acid per kg of body weight.

Inflammatory disorders of the digestive tract affect animals as well as humans. Inflammatory bowel disease is the most common cause of chronic vomiting and diarrhoea in dogs. (E.de Papp, "Inflammatory Bowel Disease in dogs", viewed 26th October 2011, http://www.petplace.com). It has also been estimated that about one-third of dogs with a history of chronic diarrhoea have colitis. (The Merck Veterinary Manual, 9th Edition). Chronic diarrhea in horses can be caused by inflammatory conditions involving the intestine. Because of the large volume of the colon and cecum of horses, massive fluid losses can occur in a short time. Thus, diarrhea in adult horses can be an explosive event with morbidity and mortality exceeding that associated with diarrheal diseases in other animals and humans (The Merck Veterinary Manual, 9th Edition). It is therefore an advantage to provide a composition for the treatment or prevention of inflammatory disorders of the digestive tract to be administered to humans or animals. In the case of companion animals, such therapies improve the animal's overall quality of life and improve owner satisfaction. The present invention provides a composition to be administered to humans, pets or livestock.

4-oxo-2-pentenoic acid and the composition described in the present invention may be administered to stressed subjects, in particular adults. This is advantageous because symptoms of digestive tract inflammation are often triggered by stress, and stress can augment the response of an inflammatory stimulus to the digestive tract (S.M. Collins, American Journal of Physiology - Gastrointestinal and Liver Physiology, 280, G315-G318 (2001)). As people age, relaxing after a stressful event becomes more difficult. A subject is considered adult if they are of relatively mature age. Typically subjects are considered adult when they are sexually mature and capable of reproduction.

4-oxo-2-pentenoic acid and the composition described in the present invention may be administered to children or adolescents. The term "children" here refers to those between birth and the beginning of sexual maturation (puberty), while "adolescents" are between puberty and adulthood. Gastroenteritis is the most common digestive disorder among children and adolescents. About 1 billion episodes occur worldwide each year, most commonly in developing countries among children under 5 years of age. Most people who develop Crohn's disease do so before age 35, usually between the ages of 15 and 25. Ulcerative colitis may start at any age but usually begins between the ages of 15 and 30 (The Merck Manual of Diagnosis and Therapy - 19th edition). Accordingly it is advantageous to have a composition for use in the treatment or prevention of inflammatory disorders of the digestive tract which may be administered to children or adolescents.

Children may be people at the age of at least 5, at least 7, at least 10 or at least 12 years old.

Adolescents may be people at the age of at least 13 or at least 14 years old.

Adults may be people at the age of at least 16, at least 18 or at least 21 years old.

The nature of the composition is not particularly limited. It may be a composition for oral or enteral administration. The composition may be for example selected from the group consisting of a food composition, a food additive, a

nutraceutical, a drink, a nutritional formulation, a tube feeding formulation, a powdered composition to be reconstituted in milk or water, and a pet food composition. Within the scope of the present invention the term nutraceutical refers to a food stuff, as a fortified food or dietary supplement, that provides a health benefit.

The composition may be a food composition. Food compositions according to the present invention are diverse in character, for example: milk, yogurt, cheese, fermented milks, milk-based fermented products, ice-creams, cereal-based products or fermented cereal-based products, milk-based powders, chilled or shelf stable beverages, confectionery, animal feed, in particular for domestic animals.

The food composition may also further comprise a protein source, a carbohydrate source, a lipid source, a mineral source and/or a vitamin source. The presence of proteins, carbohydrates, lipids, minerals and/or vitamins may have several advantages. These compounds generally contribute to the taste and mouthfeel of the final product. They also provide the body with nutrients that it may need urgently when it is affected by digestive tract disorders. They also allow formulating the composition of the present invention as a complete nutritional formula, so that no additional nutrition is needed.

Compounds soluble in water have the advantage of being conveniently administered in a number of ways, including orally as solutions, or in capsules or tablets. The composition comprising 4-oxo-2-pentenoic acid may be water-based, for example the composition may comprise 4-oxo-2-pentenoic acid dissolved in water.

Further advantages and features of the present invention are apparent from the following figures and examples.

Figure 1 shows normalized luciferase activities of crude preparations (OD 50, heated for 6 hours at 90°C) of *Bifidobacterium breve* ATCC 15700™. The results are expressed on the y-axis as a mean ± SD of technical triplicates. The x-axis values are the final dilutions of the sample.

Figure 2 shows normalized luciferase activities of crude preparations (OD 50, heated for 6 hours at 90°C) of *Bifidobacterium breve* CNCM 1-3865. The results are expressed on the y-axis as a mean ± SD of technical triplicates. The x-axis values are the final dilutions of the sample.

Figure 3 shows Nrf2 induction-fold (bars) and percentage of cell viability (lines) of AREc32 cells incubated with of a dose range of 4-oxo-2-pentenoic acid from 0 to 200 μM. The Nrf2 fold inductions are ratios between the luciferase activity (RLU) of the AREc32 cells in the presence of 4-oxo-2-pentenoic acid and the basal luciferase activity of the unexposed cells. The cell viability, measured by ATP quantification, is expressed as relative percentages of control (untreated) cells. The results are expressed as means of technical triplicates ± SD and are representative of four independent experiments.

Figure 4 shows Nrf2 induction-fold (bars) and percentage of cell viability (lines) of AREc32 cells incubated with of a dose range of a "pure preparation" of *Bifidobacterium breve* CNCM 1-3865 from 2.5 to 50% v/v. Other details as for figure 3.

Figure 5 shows NF-κB activity estimated by the production of SEAP (solid bars) and IL-8 (striped bars) measured in supernatant of HT-29 clone 34 cells stimulated with LPS. The cell viability is shown with a line. The cells were exposed to a dose range of 4-oxo-2-pentenoic acid. Results are expressed as means ± SD of two independent experiments performed in technical triplicates.

Figure 6 shows NF-κB activity (bars) and cell viability (lines) of LPS stimulated (0.5 μg/ml) THP-1 blue cells in a presence of a dose range of 4-oxo-2-pentenoic acid for 24 h. Results are expressed as means ± SD of two independent experiments performed in technical triplicates.

Figure 7 shows a typical chromatogram of a 4-oxo-2-pentenoic acid standard dissolved in water. The higher SRM is associated to the transition reaction of m/z 113→69, while the lower SRM corresponds to transition reaction of m/z 113→41. The retention time is expressed in minutes (x-axis). Signal intensity (y-axis) is expressed in Cps.

Figure 8 shows 4-oxo-2-pentenoic acid quantification using HPLC-ESI-MS/MS of crude preparations of *Bifidobacterium breve* CNCM 1-3865 (OD 40) heated for 2, 15, 30, and 60 minutes at 90°C (indicated by circles ○), 120°C (indicated by triangles Δ) and 140°C (indicated by squares □).

Example 1: Nrf2 activation by 4-oxo-2-pentenoic acid and bacterial fractions.

*Nrf2 Reporter Assay:*

Activation of Nrf2 was measured using an Nrf2 reporter assay. This assay is based on the AREc32 cell line, from CRX biosciences (Dundee, Scotland), a stably transfected MCF7 (breast adenocarcinoma) cell line that contains a luciferase gene construct under the control of the ARE. Luciferase is an enzyme which digests luciferin and produces fluorescence. Anti-oxidative molecules such as Tert-butylhydroquinone (TBHQ) induce luciferase transcription via the activation of Nrf2 that binds to ARE. Luciferase activity is determined using a luciferase kit form Promega (Madison, WI). The luciferase activity is proportional to the activation of Nrf2.

*Nrf2 activation by bacterial fractions:*

Three bacterial strains were used to investigate activation of Nrf2 by microorganisms: *Bifidobacterium breve* CNCM 1-3865 (NCC2950), *Bifidobacterium breve* CNCM 1-3914 (NCC466) and *Bifidobacterium breve* ATCC 15700™ (NCC2791). *Bifidobacterium breve* CNCM 1-3914 was deposited with the COLLECTION NATIONALE DE CULTURES DE MICROORGANISMES (CNCM), INSTITUT PASTEUR, 25 rue du Docteur Roux, F-75724 PARIS Cedex 15, France, on February 5th, 2008.

For each strain, 10 ml of MRS agar with 0.05 % cystein was inoculated with 20 μl of glycerol stock and incubated overnight at 37°C in anaerobic condition to form pre-cultures. Further cultures were then made by inoculating 10 ml of MRS with 0.05% cystein with the pre-cultures (final $OD_{600}$ adjusted at 0.1). The cultures were incubated for 16 hours at 37°C in anaerobic conditions to form the P2 cultures. 200 ml of MRS with 0.05% cystein was inoculated with the P2 cultures (final $OD_{600}$ adjusted at 0.1) and the bottles were incubated for 16 hours at 37°C in anaerobic conditions.

The $OD_{600}$ was measured, the cultures were centrifuged at 3300 g for 10 min and the bacterial pellets were washed two times with cold 1X PBS (phosphate buffered saline) and normalized to OD 50 with 1X PBS.

Bacterial fractions were obtained in two ways for each bacterial strain; a "crude preparation" and a "pure preparation".

The bacterial "crude preparations" were obtained as follows. 5 ml of the OD 50 bacterial preparations were heated for 6 hours at 90°C in a heating block (Dri-Block DB-3 heating block from Techne, Staffordshire, United Kingdom). The heated bacterial preparations were centrifuged at 3300 g for 10 min at +4°C and the supernatants were filtered using 0.22 μm syringe filters and stored at +4°C until further analyses.

The bacterial "pure preparations" were obtained as follows. 5 ml of the OD 50 bacterial preparations were centrifuged at 3300 g for 10 min at +4°C and the bacterial pellets were re-suspended with 5 ml of water. The bacterial cells were disrupted using mini bead beat (MBB) apparatus in a cold room (six cycles of 90 sec at maximum speed with 10 min of pause between each cycle). The disrupted cells were centrifuged for 1 h at 3300 g at +4°C and the pellet was re-suspended with 5 ml of 1X PBS and heated for 6 hours at 90°C in a heating block. The heated preparations were centrifuged for 10 min at 3300 g at +4°C. The supernatants were filtered using 0.22 μm syringe filters and stored at +4°C until further analyses.

The live bacteria counts of the "OD 50 suspensions" were determined by plating using a spotting method, and the dry weights determined using a halogen moisture analyzer (Metler-Toledo, Greifensee, Switzerland) with the following settings: drying temperature 160°C with step-drying activated.

To determine the Nrf2 activation the samples were tested on AREc32 cells (seeded in 96 well plates) using 10 independent dilutions (1/2, 1/4, 1/6, 1/10, 1/15, 1/20, 1/25, 1/30, 1/40 and 1/50) and incubated for 24 hours at 37°C in a 5% CO2/air incubator. The luciferase activity and the cell viability (ATP measurements) were determined using the Luciferase and Cell Titer-Glo kits from Promega.

For each run the luciferase activities, measured in Relative Light Units (RLU), of all the wells were normalized with the mean of the luciferase activity of the cells only of all the plates. Among all the samples tested the normalization procedure was found not to affect the data and this observation permits the comparison of samples measured in different runs.

For each sample the Nrf2 activation was calculated as follows:

1) The Nrf2 fold induction:

$$Nrf2\ fold\ induction = \frac{Normalized\ luciferase\ activity\ of\ the\ sample}{Normalized\ luciferase\ activity\ of\ the\ cells}$$

The Nrf2 fold induction is very useful for screening purposes.

However the Nrf2 fold induction is a qualitative measurement only, because this calculation does not take into account the sample dilution.

2) The luciferase content per sample:

$$\begin{pmatrix}\text{Luciferase content}\\ \text{per sample}\end{pmatrix} = \begin{pmatrix}\text{Normalized luciferase activity}\\ \text{of the sample dilution giving the}\\ \text{highest Nrf2 fold induction}\end{pmatrix} \text{ x } \begin{pmatrix}\text{Dilution factor}\\ \text{giving the highest}\\ \text{Nrf2 fold induction}\end{pmatrix}$$

The "luciferase content per sample" also reflects Nrf2 activation but can differentiate two samples activating Nrf2 at similar Nrf2 fold inductions since this calculation takes into account the sample dilution.
The luciferase content per sample allows a semi quantification of the Nrf2 activation by reflecting the amount of the Nrf2 activating molecule.

Table A: Normalized luciferase activities and calculation of "luciferase content per sample" for crude preparations (OD 50, heated for 6 hours at 90°C) of Bifidobacterium breve ATCC 15700™

| ***Bifidobacterium breve* ATCC 15700** | | | | | | | | | | |
|---|---|---|---|---|---|---|---|---|---|---|
| Normalized luciferase content of the cells = 9583 | | | | | | | | | | |
| Sample dilution | 1/2 | 1/4 | 1/6 | 1/10 | 1/15 | 1/20 | 1/25 | 1/30 | 1/40 | 1/50 |
| Normalized luciferase | 9.37E5 | 3.11E5 | 8.02E4 | 3.28E4 | 2.15E4 | 1.72E4 | 1.54E4 | 1.52E4 | 1.41E4 | 1.32E4 |
| Dilution factor | 2 | 4 | 6 | 10 | 15 | 20 | 25 | 30 | 40 | 50 |
| Nrf2 fold induction | 97.8 | 32.5 | 8.4 | 3.4 | 2.2 | 1.8 | 1.6 | 1.6 | 1.5 | 1.4 |
| Luciferase content per sample = 9.37E5 x 2 = 1.87E6<br>(The scientific notation 9.4E5 is equivalent to 9.4 x $10^5$) | | | | | | | | | | |

Table B: Normalized luciferase activities and calculation of "luciferase content per sample" for crude preparations (OD 50, heated for 6 h. at 90°C) of Bifidobacterium breve CNCM 1-3865

| ***Bifidobacterium breve* CNCM I-3865** | | | | | | | | | | |
|---|---|---|---|---|---|---|---|---|---|---|
| Normalized luciferase content of the cells = 9583 | | | | | | | | | | |
| Sample dilution | 1/2 | 1/4 | 1/6 | 1/10 | 1/15 | 1/20 | 1/25 | 1/30 | 1/40 | 1/50 |
| Normalized luciferase | 1.16E3 | 1.46E3 | 2.62E3 | 1.24E4 | 1.05E6 | 5.19E5 | 1.04E6 | 8.30E5 | 3.37E5 | 1.87E5 |
| Dilution factor | 2 | 4 | 6 | 10 | 15 | 20 | 25 | 30 | 40 | 50 |
| Nrf2 fold induction | 0.1 | 0.2 | 0.3 | 1.3 | 10.9 | 54.1 | 108.3 | 93.4 | 35.1 | 19.5 |
| Luciferase content per sample = 1.04E+06 x 25 = 2.60E+07 | | | | | | | | | | |

As illustrated in tables A and B, both crude preparations of *B. breve* ATCC 15700™ and *B. breve* CNCM 1-3865 have similar maximum Nrf2 induction but different luciferase content/sample values. The difference in luciferase content per sample values reflect their corresponding Nrf2 activation patterns (see figures 1 and 2).

In contrast *Bifidobacterium breve* CNCM 1-3914 did not significantly activate Nrf2, see comparison table C.

Table C: Comparison of results from the three different *Bifidobacterium breve* strains - crude preparation.

| B. breve strain code | cfu/ml | Dry weight (mg/ml) | Normalized luciferase activity | Dilution factor giving the highest Nrf2 fold induction | Nrf2 fold induction | Luciferase content per sample |
|---|---|---|---|---|---|---|
| CNCM 1-3865 | 2.5E10 | 24.8 | 1.04E6 | 25 | 108.3 | 2.60E7 |
| ATCC 15700 | 1.7E10 | 23.2 | 9.37E5 | 2 | 97.8 | 1.87E6 |
| CNCM 1-3914 | 1.8E10 | 24.1 | 3.06E4 | 2 | 3.2 | 6.12E4 |

Table D: Comparison of results from the three different Bifidobacterium breve strains - pure preparation.

| B. breve strain code | cfu/ml | Dry weight (mg/ml) | Normalized luciferase activity | Dilution factor giving the highest Nrf2 fold induction | Nrf2 fold induction | Luciferase content per sample |
|---|---|---|---|---|---|---|
| CNCM 1-3865 | 2.5E10 | 24.8 | 8.00E5 | 40 | 106 | 3.20E7 |
| ATCC 15700 | 1.7E10 | 23.2 | 6.55E5 | 6 | 68.4 | 3.93E6 |
| CNCM 1-3914 | 1.8E10 | 24.1 | 2.42E4 | 2 | 2.5 | 4.84E4 |

*Nrf2 activation by 4-oxo-2-pentenoic acid*

4-oxo-2-pentenoic acid (Alfa Aesar - reference L02185) was tested in the Nrf2 reporter assay. Different doses of 4-oxo-2-pentenoic acid were applied on AREc32 cells for 24 hours and then the luciferase activity was quantified as described above. The cell viability was also measured using a cell Titer-Glo kit (ATP quantification).

As shown in figure 3, the 4-oxo-2-pentenoic acid molecule was found to strongly activate Nrf2 in a dose dependent manner. The viability of AREc32 cells was not affected by 4-oxo-2-pentenoic acid at doses lower than 70 $\mu$M. The optimal dose of Nrf2 activation was around 40-50 $\mu$M. For comparison, figure 4 shows that a bacterial fraction of *Bifidobacterium breve* CNCM 1-3865 activates Nrf2 in a similar manner.

Example 2: Inhibition NF-$\kappa$B activation by 4-oxo-2-pentenoic acid (anti-inflammatory effect).

The inventors evaluated the capability of 4-oxo-2-pentenoic acid to inhibit NF-$\kappa$B activation under pro-inflammatory stresses (LPS and rhTNF-$\alpha$). Two in-vitro systems were used: human colonic cells (HT-29 clone 34) and human monocytes/macrophages (THP-1 blue cells).

*HT-29 clone 34*

The human colonic adenocarcinoma HT-29 clone 34 cell line (passage 42-50) are adherent cells stably transfected with a NF-$\kappa$B/SEAP reporter plasmid. They were cultured in DMEM high glucose (4.5 g/L) (Invitrogen) containing 1% of stable L-glutamine and supplemented with 10% of heat inactivated (one hour at 56°C) Foetal Calf Serum (FCS) (Bioconcept, Allschwil, Switzerland), 1% of Penicillin/Streptomycin (Sigma) and 500 $\mu$g/ml of Geneticin (PAA, Pasching, Austria) at 37°C in a 5% $CO_2$/air incubator. Culture medium was renewed every two days until the cell monolayer reached ~ 90 % confluence. Cells were sub-cultured using 1X Trypsin/EDTA (Sigma).

10000 cells/well were seeded in 200 $\mu$l of culture medium in flat bottom white border 96 well plates (Greiner

Bio One, Kremsmuenster, Austria). After 3-4 days of culture (i.e. cells reaching ~ 70-80 % confluence), culture medium was removed and 180 μl of experimental medium (DMEM high glucose supplemented with 50 mM of HEPES and 5% of human milk (HM), as a source of sCD14 and LPS Binding Protein (LBP), for LPS stimulation only) containing (or not) dose ranges of 4-oxo-2-pentenoic acid (3 to 400 μM) were added to the cells and the plates were pre-incubated for 4 hours at 37°C in a 5% $CO_2$/air incubator. 20 μl of experimental medium containing (or not) LPS 055:B5 or rhTNF-α (100 and 10 ng/ml final, respectively) was added and the plates were incubated for 24 hours at 37°C in a 5% $CO_2$/air incubator.

Cell culture supernatants were then collected for measurement of NF-κB activity (determination of secreted alkaline phosphatase and IL-8 production) using Phosphalight (Applied Biosystems) and IL-8 singleplex (Meso Scale, Gaithersburg, Maryland) kits, respectively.

Cell viability was determined by measuring ATP using a Cell Titer-Glo kit (Promega). Briefly, remaining adherent HT-29 clone 34 cells were incubated for 10 min at room temperature under horizontal shaking (250 rpm) with 120 μl of Cell Titer-Glo reagent (pre-diluted twice in 1X PBS) and the luminescence was measured using Polarstar microplate reader (BMG, Offenburg, Germany) for 1000 ms with a gain value set to 3500.

In the absence of LPS no NF-κB activity was observed (based on SEAP or IL-8 detection) in cell culture supernatants of HT-29 clone 34 cells incubated with 4-oxo-2-pentenoic acid at the doses tested.

Measurements of the cell viability of the HT-29 clone 34 cells showed a decrease in cell viability at doses of 4-oxo-2-pentenoic acid higher than ~ 50 μM (figure 5).

When inflammation response is trigged by LPS, 4-oxo-2-pentenoic acid inhibits NF-κB activity in a dose dependent manner (figure 5). 4-oxo-2-pentenoic acid impacted on both SEAP and IL-8 secretion, the best inhibition being with 50 μM 4-oxo-2-pentenoic acid). Similar results, although less pronounced, were observed with cells stimulated with rhTNF-α.

*THP-1 blue*

Human monocytes/macrophages THP-1 blue cells (passage 16-20) (Invivogen, Toulouse, France) were cultured in modified RPMI medium (ATCC, Manassas, VA) containing 1 mM of sodium pyruvate, 2 mM of L-glutamine, 4.5 g/L of glucose and 10 mM of HEPES supplemented with 10 % of heat inactivated FCS (Bioconcept), 1 % of Penicillin/Streptomycin (Sigma) and 500 μg/ml of Zeocin (Invivogen) at 37°C in a 5% $CO_2$/air incubator.

200000 cells/well were seeded in 100 μl of culture medium in 96 well flat bottom transparent plates. After 24 hours of incubation at 37°C in a 5% $CO_2$/air incubator, 80 μl of culture medium containing (or not) dose ranges of 4-oxo-2-pentenoic acid (5 to 200 μM) were added to the cells and pre-incubated for 5 hours at 37°C. 20 μl of culture media containing (or not) LPS 055:B5 (Sigma) (100 ng/ml final) was added and the plates were incubated for 16 hours at 37°C in a 5% $CO_2$/air incubator.

Cell culture supernatants were carefully transferred to 96 well-plates for NF-κB activity measurement which is proportional to the level of secreted alkaline phosphatase. Briefly, 100 μl of supernatants were mixed with 150 μl of QuantiBlue (Invivogen) in a 96 well-plates incubated for 3 h at 37°C before OD measurements at 620 nm with Sunrise microplate reader (Tecan, Mannedorf, Switzerland). Cell viability was determined using Cell Titer-Glo kit as described above.

When stimulated with LPS, strong NF-κB inhibition was obtained in response to 4-oxo-2-pentenoic acid at doses around 40 to 75 μM. 4-oxo-2-pentenoic acid was toxic to cells at doses higher than 75 μM (figure 6).

The data generated with HT-29 clone 34 cells and THP-1 blue cells indicate that 4-oxo-2-pentenoic acid inhibits NF-κB activation under pro-inflammatory stresses.

<u>Example 3: Quantification of 4-oxo-2-pentenoic acid by HPLC-MS/MS.</u>

In order to quantify 4-oxo-2-pentenoic acid, a high throughput analytical method involving coupling high performance liquid chromatography with electrospray ionization tandem mass spectrometry (HPLC-ESI-MS/MS) was developed.

4-oxo-2-pentenoic acid standard was purchased from Alfa Aesar (Ward Hill, USA). 4-oxo-2-pentenoic acid was found to be soluble in water to at least 20 mg/ml. 4-oxo-2-pentenoic acid standard compound was solubilised in water at a final stock solution of 10 mg/ml and further diluted in water to build a calibration curve.

HPLC-ESI-MS/MS analyses were carried out on a turbulent flow chromatography (TFC) system (Thermo Fisher, Waltham, MA) coupled to a 3200 Q TRAP mass spectrometer (Applied Biosystems). The analytical column used was a Hypersil Gold AQ (3 x 50 mm, 5 μm) purchased from Thermo Fisher (Waltham, MA) running at room temperature and a constant flow rate of 600 μl/min. The mobile phases were constituted with solvent A - water containing 0.05% acetic acid and B - methanol containing 0.05% acetic acid. The gradient program was: 0 min 0% B, held for 40 sec (0-0.67 min) at 0% B, ramping to 50% B in 180 sec (0.67-3.67 min), ramping from 50 to 90% B in 10 sec (3.67-3.83 min), held for 120 sec (5.83 min) at 90% B, before going back to 0% B and held for an additional 300 sec (5.83-10.83 min). The

injection volume was 5 μl.

MS data acquisition was realized in electrospray negative ionization mode. MS tuning was performed by infusing a solution of 4-oxo-2-pentenoic acid standard (5 μg/ml in water) at a flow rate of 10 μl/min mixed with a HPLC flow made of solvents A and B (80/20, v:v; 0.6 ml/min) using a T-connector. Nitrogen was used for the nebulizer and curtain gases at pressures of respectively 70, 30 and 20 psi. The interface heater was activated and the block source temperature was maintained at 700°C with a capillary voltage set at -4.5 kV. Nitrogen was also used as collision gas at a medium pressure selection. MS/MS detection was realized using the selected reaction monitoring (SRM) acquisition mode. The two most intense fragment ions were selected by scanning m/z 113→69 (collision energy of 11 eV), and m/z 113→41 (collision energy of 26 eV), using a constant dwell times of 50 ms (total scan time of 110 ms). The declustering potential was set at -29 V. Quantitative analyses were performed using the most intense SRM signal whereas the second transition was used for analyte confirmation based on appropriate area ratio calculated from standard solutions. Data processing was performed using Analyst 1.5.1 software (Applied Biosystems).

*Detection of 4-oxo-2-pentenoic acid in PBS and water by HPLC-MS/MS:*

4-oxo-2-pentenoic acid was solubilised in 1X PBS or water, and the detection by HPLC-MS/MS performed as described in the previous section. The SRM associated to the transition reaction of m/z 113→69 revealed a more intense signal as compared to the SRM associated with the transition m/z 113→41 at a retention time of 1.25 min. Similar retention time for both SRMs were observed confirming the validity of the analysis (figure 7). The molecule 4-oxo-2-pentenoic acid was successfully detected in both 1X PBS (data not shown) and water (figure 7).

*Establishment of 4-oxo-2-pentenoic acid standard curve:*

In order to quantify precisely the amount of 4-oxo-2-pentenoic acid in bacterial fractions, standard curves were established for 4-oxo-2-pentenoic acid in simple matrices like 1X PBS or HPLC grade water. Commercial 4-oxo-2-pentenoic acid was suspended in 1X PBS and water at different doses. The HPLC-ESI-MS/MS method was then used to quantify the estimated doses of 4-oxo-2-pentenoic acid. Good linearity was observed between the quantity of 4-oxo-2-pentenoic acid (from 0.1 to 25 μg/ml) and the resulting intensities (expressed in cps) both in 1X PBS and HPLC grade water.

*Quantification of 4-oxo-2-pentenoic acid in bacterial fractions:*

4-oxo-2-pentenoic acid was quantified in the heat treated bacterial preparations from example 1. All samples were diluted in HPLC grade water (3 dilutions/sample) before HPLC-ESI-MS/MS analysis. The results are summarized in table E.

Table E: Concentrations of 4-oxo-2-pentenoic acid (μg/ml) in crude and pure bacterial heated preparations (OD 50) from example 1 (6 hours of heating at 90°C). N.D stands for "Not Detectable", below the detection limit of the method.

| Strain | Strain Code | 4-oxo-2-pentenoic acid (μg/ml) Crude preparation | 4-oxo-2-pentenoic acid (μg/ml) Pure preparation |
|---|---|---|---|
| B. breve | CNCM 1-3865 | 95.3 | 126.8 |
| B. breve | ATCC 15700 | 2.1 | 16.4 |
| B. breve | CNCM 1-3914 | N.D. | N.D. |

<u>Example 4: The influence of heating temperature and time on the production of 4-oxo-2-pentenoic acid from *Bifidobacterium breve* CNCM I-3865</u>

To characterize the production of 4-oxo-2-pentenoic acid from *Bifidobacterium breve* CNCM 1-3865 upon heat treatment a kinetic experiment was performed using various temperatures. The "master stock" of biomass used for this experiment was produced in bioreactors at 37°C with MRS medium under anaerobic and pH control conditions. After the grow culture (16h), the culture media was removed and the cells were washed two times with 1X PBS, concentrated to OD 134 (1.5E+10 cfu/ml) in 1X PBS with 10% glycerol then stored at -80°C in 50 ml aliquots.

A "working biomass" of *Bifidobacterium breve* CNCM 1-3865 was then prepared from the biomass master stock as follows: The biomass was washed two times with 1X PBS and adjusted to OD 40 in 1X PBS.

A Temperature Heating Apparatus (THA) was used to investigate the effect of different heating times and

temperatures. This system is a small scale version of typical apparatus found in production environments. Steam is used to heat up a holding tube containing cartridges of biomass. Sample temperatures of 90°C, 120°C and 140°C were applied for periods up to 60 minutes. 5 ml of each heat-treated biomass was then centrifuged for 10 min at 5000 g and the supernatants were filtered (0.2 μm) and the 4-oxo-2-pentenoic acid content quantified by HPLC-ESI-MS/MS. The amounts of 4-oxo-2-pentenoic acid generated are shown in figure 8.

## Claims

**1.** Composition comprising 4-oxo-2-pentenoic acid for use in the treatment or prevention of inflammatory disorders of the digestive tract selected from the group consisting of ileitis, colitis, pharyngitis, leaky gut syndrome, irritable bowel syndrome, rectal inflammation, inflammatory bowel disease and combinations thereof; wherein the composition is not a pharmaceutical.

**2.** Composition for use in accordance with claim 1, wherein the 4-oxo-2-pentenoic acid is obtained from natural sources.

**3.** Composition for use in accordance with one of the preceding claims, wherein the 4-oxo-2-pentenoic acid is obtainable from *Bifidobacterium breve* CNCM 1-3865 or *Bifidobacterium breve* ATCC 15700.

**4.** Composition for use in accordance with claim 3, wherein the *Bifidobacterium breve* CNCM 1-3865 or *Bifidobacterium breve* ATCC 15700 was heat treated at about 60-180°C, preferably at about 80-160°C.

**5.** Composition for use in accordance with one of the preceding claims comprising 4-oxo-2-pentenoic acid in an amount of at least 1 mg per kg of the composition, preferably at least 10 mg per kg of the composition, for example between 50 mg and 50 g per kg of the composition.

**6.** Composition for use in accordance with one of the preceding claims to be administered in a daily dose corresponding to between 2 μg and 20 mg of 4-oxo-2-pentenoic acid per kg of body weight, preferably between 20 μg and 2 mg of 4-oxo-2-pentenoic acid per kg of body weight, for example between 40 μg and 1 mg of 4-oxo-2-pentenoic acid per kg of body weight.

**7.** Composition for use in accordance with one of the preceding claims to be administered orally or enterally.

**8.** Composition for use in accordance with one of the preceding claims to be administered to humans, pets or livestock.

**9.** Composition for use in accordance with one of the preceding claims to be administered to stressed subjects, in particular adults.

**10.** Composition for use in accordance with one of the preceding claims to be administered to children or adolescents.

**11.** Composition for use in accordance with one of the preceding claims wherein the composition is selected from the group consisting of a food composition, a food additive, a nutraceutical, a drink, a nutritional formulation, a tube feeding formulation, a powdered composition to be reconstituted in milk or water, and a pet food composition.

## Patentansprüche

**1.** Zusammensetzung, umfassend 4-Oxo-2-pentensäure zur Verwendung bei der Behandlung oder Prävention von entzündlichen Erkrankungen des Verdauungstraktes, ausgewählt aus der Gruppe bestehend aus Ileitis, Colitis, Pharyngitis, Leaky-Gut-Syndrom, Reizdarmsydrom, Enddarmentzündung, entzündlicher Darmerkrankung und Kombinationen davon, wobei die Zusammensetzung nicht pharmazeutisch ist.

**2.** Zusammensetzung zur Verwendung nach Anspruch 1, wobei die 4-Oxo-2-pentensäure aus natürlichen Quellen erhalten wird.

**3.** Zusammensetzung zur Verwendung nach einem der vorstehenden Ansprüche, wobei die 4-Oxo-2-pentensäure aus *8Bifidobacterium breve* CNCM I-3865 oder *Bifidobacterium breve* ATCC 15700 erhalten werden kann.

4. Zusammensetzung zur Verwendung nach Anspruch 3, wobei das *Bifidobacterium breve* CNCM I-3865 oder *Bifidobacterium breve* ATCC 15700 bei etwa 60 bis 180 °C, bevorzugt bei etwa 80 bis 160 °C, erhitzt wurde.

5. Zusammensetzung zur Verwendung nach einem der vorstehenden Ansprüche, umfassend 4-Oxo-2-pentensäure in einer Menge von mindestens 1 mg pro kg der Zusammensetzung, vorzugsweise mindestens 10 mg pro kg der Zusammensetzung, zum Beispiel zwischen 50 mg und 50 g pro kg der Zusammensetzung.

6. Zusammensetzung zur Verwendung nach einem der vorstehenden Ansprüche, die in einer täglichen Dosis, die zwischen 2 μg und 20 mg 4-Oxo-2-pentensäure pro kg Körpergewicht entspricht, vorzugsweise zwischen 20 μg und 2 mg 4-Oxo-2-pentensäure pro kg Körpergewicht, zum Beispiel zwischen 40 μg und 1 mg 4-Oxo-2-pentensäure pro kg Körpergewicht, zu verabreichen ist.

7. Zusammensetzung zur Verwendung nach einem der vorstehenden Ansprüche, die entweder oral oder enteral zu verabreichen ist.

8. Zusammensetzung zur Verwendung nach einem der vorstehenden Ansprüche, die an Menschen, Haustiere oder Nutztiere zu verabreichen ist.

9. Zusammensetzung zur Verwendung nach einem der vorstehenden Ansprüche, die an gestresste Patienten, insbesondere an Erwachsene zu verabreichen ist.

10. Zusammensetzung zur Verwendung nach einem der vorstehenden Ansprüche, die an Kinder oder Heranwachsende zu verabreichen ist.

11. Zusammensetzung zur Verwendung nach einem der vorstehenden Ansprüche, wobei die Zusammensetzung ausgewählt ist aus der Gruppe bestehend aus einer Lebensmittelzusammensetzung, einem Lebensmittelzusatz, einem nutrazeutischen Mittel, einem Getränk, einer Nährmittelzubereitung, einer Sondennahrungszubereitung, einer pulverförmigen Zusammensetzung, die in Milch oder Wasser rekonstituiert wird, und einer Haustierfutterzusammensetzung.

**Revendications**

1. Composition comprenant de l'acide 4-oxo-2-penténoïque, destinée à être utilisée dans le traitement ou la prévention de troubles inflammatoires du tube digestif choisis dans le groupe constitué par l'iléite, la colite, la pharyngite, le syndrome de perméabilité intestinale, le syndrome du côlon irritable, l'inflammation du rectum, la maladie inflammatoire de l'intestin et leurs combinaisons ; où la composition n'est pas un médicament.

2. Composition utilisable selon la revendication 1, dans laquelle l'acide 4-oxo-2-penténoïque est obtenu à partir de sources naturelles.

3. Composition utilisable selon l'une quelconque des revendications précédentes, dans laquelle l'acide 4-oxo-2-penténoïque peut être obtenu à partir de *Bifidobacterium* breve CNCM I-3865 ou de *Bifidobacterium* breve ATCC 15700.

4. Composition utilisable selon la revendication 3, dans laquelle le *Bifidobacterium* breve CNCM I-3865 ou le *Bifidobacterium* breve ATCC 15700 a été traité thermiquement à environ 60 à 180 °C, de préférence à environ 80 à 160 °C.

5. Composition utilisable selon l'une des revendications précédentes, comprenant de l'acide 4-oxo-2-penténoïque en une quantité d'au moins 1 mg par kg de la composition, de préférence au moins 10 mg par kg de la composition, par exemple entre 50 mg et 50 g par kg de la composition.

6. Composition utilisable selon l'une des revendications précédentes, destinée à être administrée dans une dose journalière correspondant à une quantité comprise entre 2 μg et 20 mg d'acide 4-oxo-2-penténoïque par kg de poids corporel, de préférence entre 20 μg et 2 mg d'acide 4-oxo-2-penténoïque par kg de poids corporel, par exemple, entre 40 μg et 1 mg d'acide 4-oxo-2-penténoïque par kg de poids corporel.

7. Composition utilisable selon l'une quelconque des revendications précédentes, destinée à être administrée par voie orale ou par voie entérale.

8. Composition utilisable selon l'une quelconque des revendications précédentes, destinée à être administrée à l'homme, aux animaux domestiques ou au bétail.

9. Composition utilisable selon l'une quelconque des revendications précédentes, destinée à être administrée à des sujets stressés, en particulier des adultes.

10. Composition utilisable selon l'une quelconque des revendications précédentes, destinée à être administrée à des enfants ou à des adolescents.

11. Composition utilisable selon l'une quelconque des revendications précédentes, dans laquelle la composition est choisie dans le groupe constitué par une composition alimentaire, un additif alimentaire, un nutraceutique, une boisson, une formulation nutritionnelle, une formulation d'alimentation par sonde, une composition en poudre à reconstituer dans du lait ou de l'eau, et une composition alimentaire pour animaux de compagnie.

FIG 1

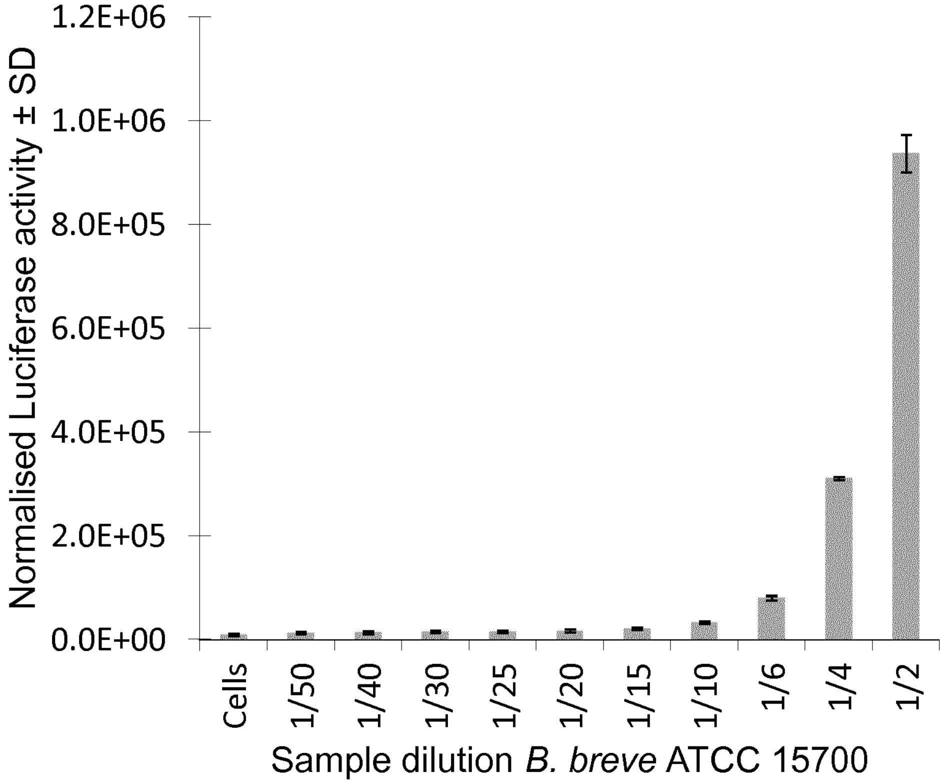
Normalised Luciferase activity ± SD
1.2E+06
1.0E+06
8.0E+05
6.0E+05
4.0E+05
2.0E+05
0.0E+00
Cells
1/50
1/40
1/30
1/25
1/20
1/15
1/10
1/6
1/4
1/2
Sample dilution B. breve ATCC 15700

FIG 2

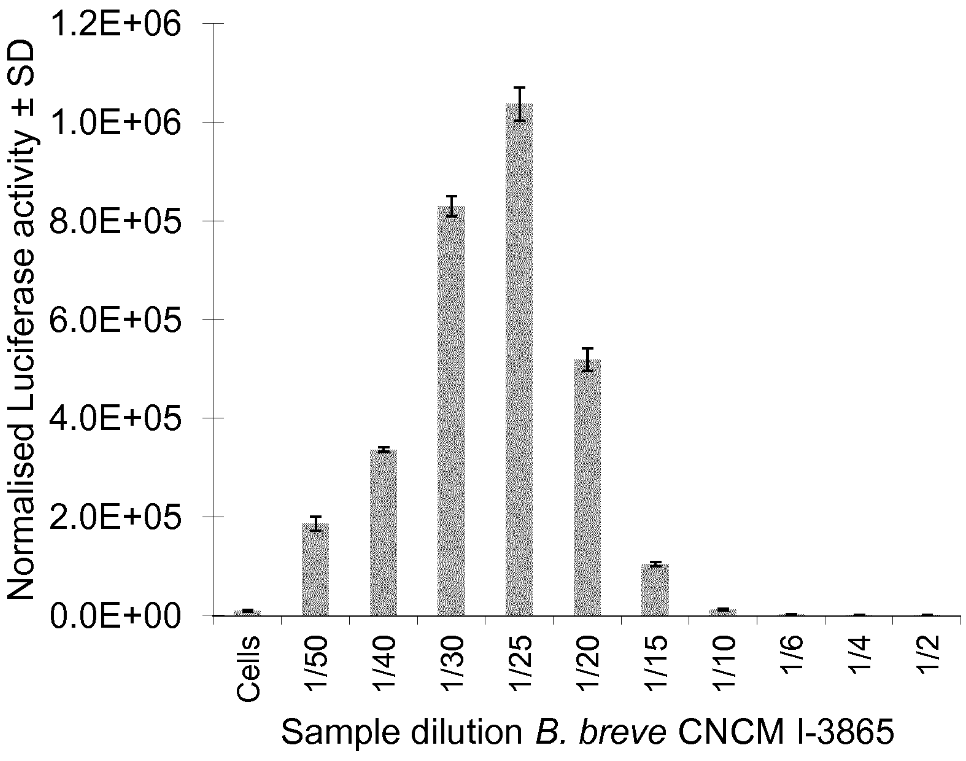
Normalised Luciferase activity ± SD
1.2E+06
1.0E+06
8.0E+05
6.0E+05
4.0E+05
2.0E+05
0.0E+00
Cells
1/50
1/40
1/30
1/25
1/20
1/15
1/10
1/6
1/4
1/2
Sample dilution B. breve CNCM I-3865

FIG 3

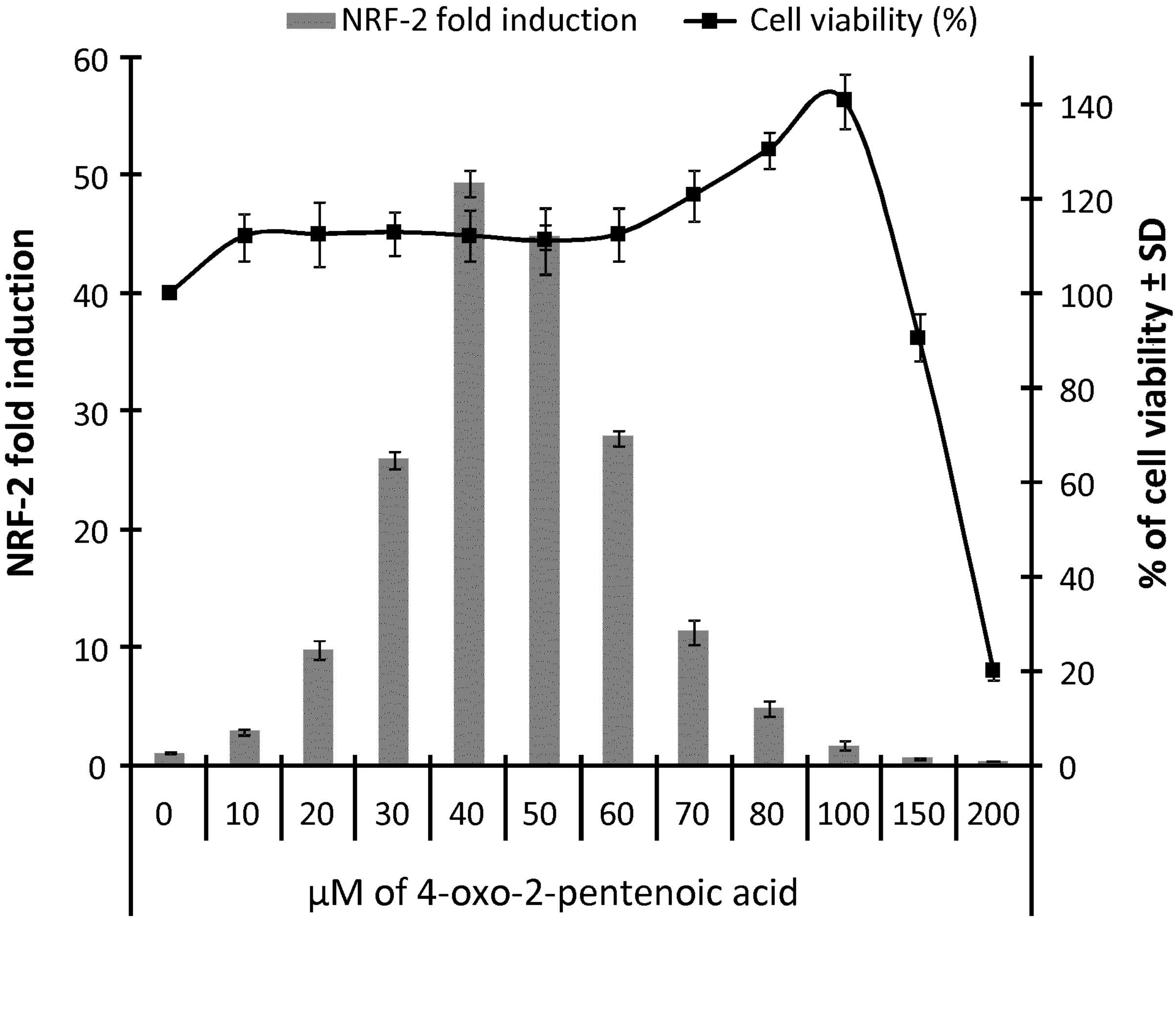
NRF-2 fold induction
Cell viability (%)
NRF-2 fold induction
% of cell viability ± SD
0
10
20
30
40
50
60
0
20
40
60
80
100
120
140
0 10 20 30 40 50 60 70 80 100 150 200
µM of 4-oxo-2-pentenoic acid

FIG 4

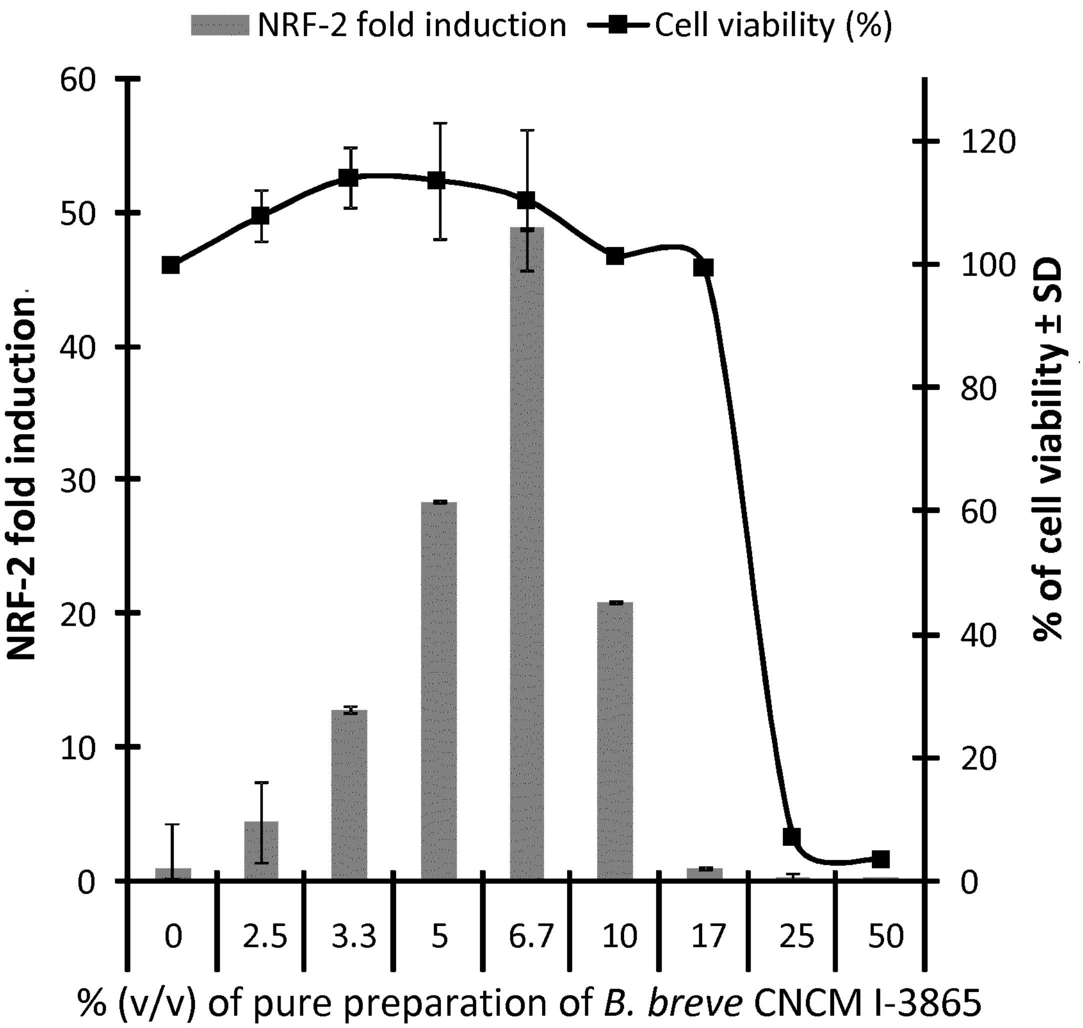
NRF-2 fold induction
Cell viability (%)
60
50
40
30
20
10
0
NRF-2 fold induction
120
100
80
60
40
20
0
% of cell viability ± SD
0
2.5
3.3
5
6.7
10
17
25
50
% (v/v) of pure preparation of *B. breve* CNCM I-3865

FIG 5

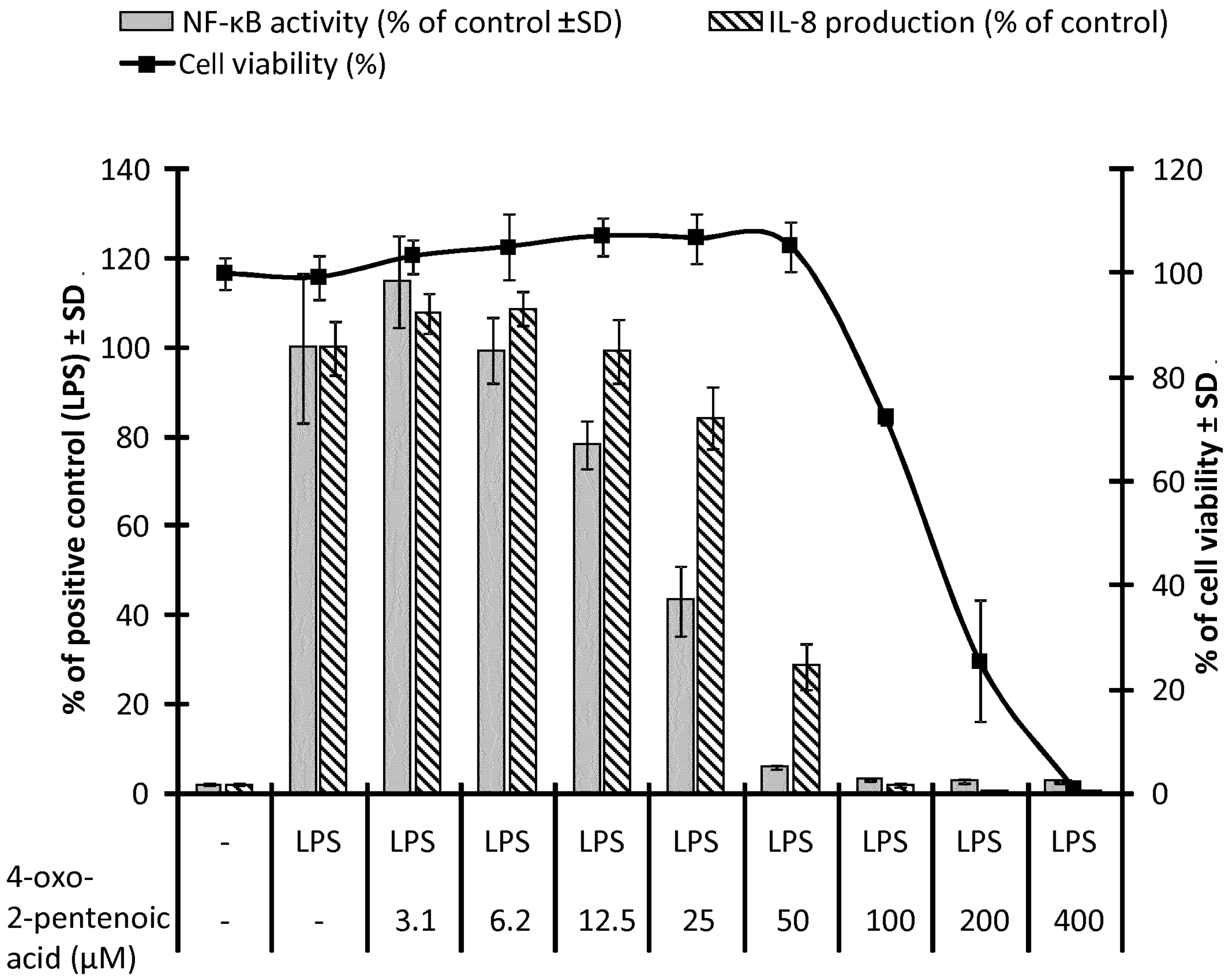
NF-κB activity (% of control ±SD)
IL-8 production (% of control)
Cell viability (%)
% of positive control (LPS) ± SD
% of cell viability ± SD
140
120
100
80
60
40
20
0
120
100
80
60
40
20
0
- LPS LPS LPS LPS LPS LPS LPS LPS LPS
4-oxo-
2-pentenoic
acid (µM)
- - 3.1 6.2 12.5 25 50 100 200 400

FIG 6

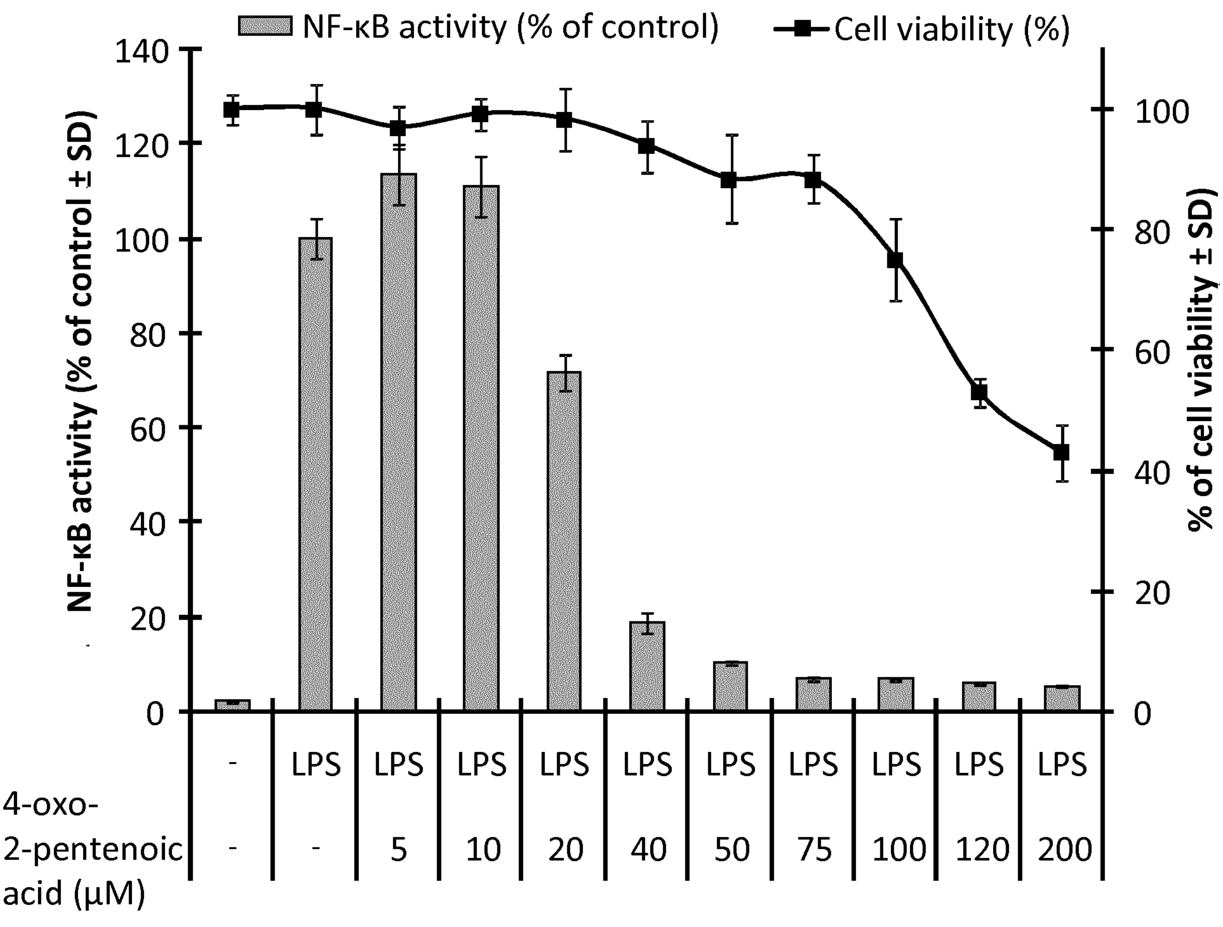
NF-κB activity (% of control)
Cell viability (%)
NF-κB activity (% of control ± SD)
% of cell viability ± SD
140
120
100
80
60
40
20
0
100
80
60
40
20
0
- LPS LPS LPS LPS LPS LPS LPS LPS LPS LPS
4-oxo-2-pentenoic acid (μM)
- - 5 10 20 40 50 75 100 120 200

FIG 7

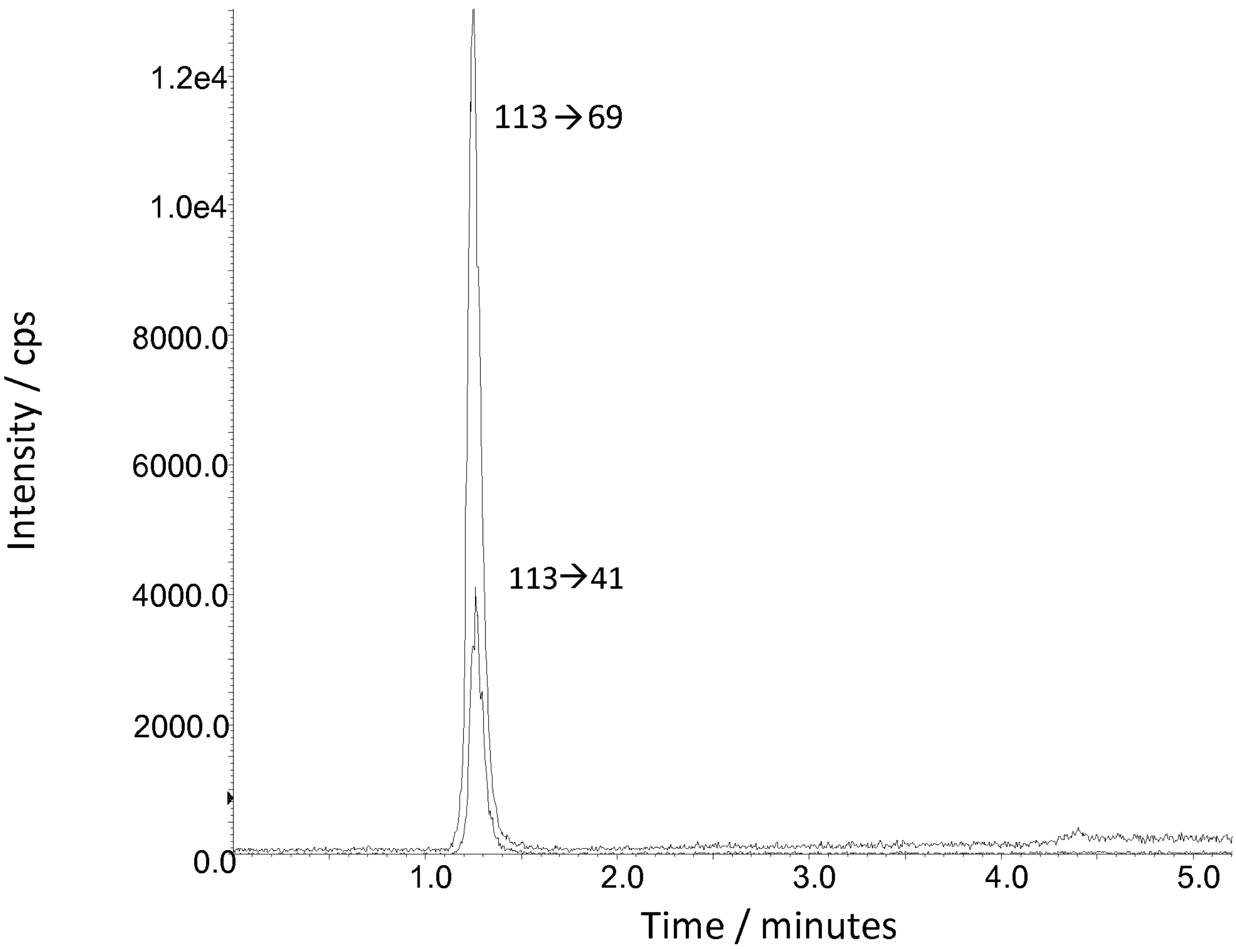
1.2e4
1.0e4
8000.0
6000.0
4000.0
2000.0
0.0
Intensity / cps
113→69
113→41
1.0
2.0
3.0
4.0
5.0
Time / minutes

FIG 8

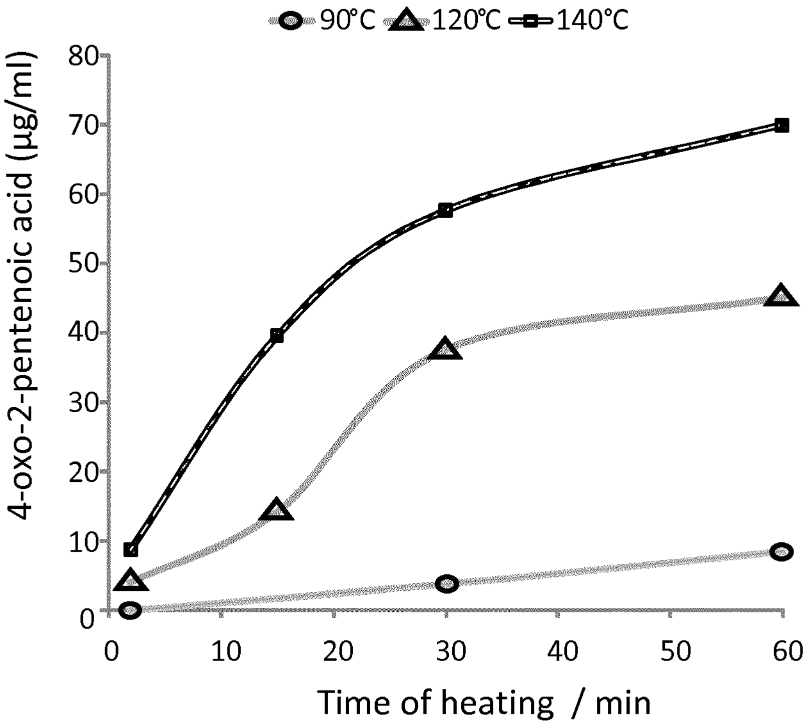
90°C
120°C
140°C
4-oxo-2-pentenoic acid (µg/ml)
0
10
20
30
40
50
60
70
80
0
10
20
30
40
50
60
Time of heating / min

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090042980 A
- WO 200119376 A
- WO 9313076 A


### Non-patent literature cited in the description


- **C.L.L. SAW et al.** *Expert Opinion on Therapeutic Targets,* 2011, vol. 15, 281-295
- **KENSLER TW et al.** *Annu Rev Pharmacol Toxicol,* 2007, vol. 47, 89-116
- **J. KIM et al.** *Mutation Research - Fundamental and Molecular Mechanisms of Mutagenesis,* 2010, vol. 690, 12-23
- **CHEN CY et al.** *Biochem Biophys Res Commun,* 17 June 2005, vol. 331 (4), 993-1000
- **F. ELBARBRY et al.** *Journal of Medical Plants Research,* 2011, vol. 5, 473-484
- **TANIGAWA S et al.** *Free Radic Biol Med,* 01 June 2007, vol. 42 (11), 1690-703
- **M. PASPARAKIS.** *Mucosal Immunology,* 2008, vol. 1, S54-S57
- **ADCOCK et al.** *American Journal of Physiology - Cell Physiology,* 1995, vol. 268, C331-C338
- **B. BROM.** *South African Family Practice,* 2010, vol. 52, 314-316
- **G. BARBARA et al.** *Gut,* 2002, vol. 51 (1), i41-i44
- **E.DE PAPP.** *Inflammatory Bowel Disease in dogs,* 26 October 2011, http://www.petplace.com
- **S.M. COLLINS.** *American Journal of Physiology - Gastrointestinal and Liver Physiology,* 2001, vol. 280, G315-G318